(19) Europäisches Patentamt
European Patent Office
Office européen des brevets

(11) Veröffentlichungsnummer: **0 063 742**
**A2**

(12) # EUROPÄISCHE PATENTANMELDUNG

(21) Anmeldenummer: **82103116.8**

(22) Anmeldetag: **13.04.82**

(51) Int. Cl.³: **C 07 D 333/36**, C 07 D 333/32, A 01 N 43/10

(30) Priorität: **24.04.81 DE 3116297**

(43) Veröffentlichungstag der Anmeldung: **03.11.82 Patentblatt 82/44**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Elbe, Hans-Ludwig, Dr., Dasnöckel 59, D-5600 Wuppertal 1 (DE)**
Erfinder: **Hammann, Ingeborg, Dr., Belfortstrasse 9, D-5000 Köln 1 (DE)**

(54) **Substituierte Carbamoyloximino-tetrahydrothiophene, Verfahren zu ihrer Herstellung und ihre Verwendung als Schädlingsbekämpfungsmittel.**

(57) Die vorliegende Erfindung betrifft neue substituierte Carbamoyloximino-tetrahydrothiophene sowie deren Derivate der Formel (I)

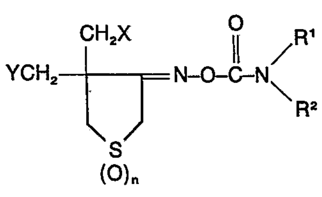

(I)

in welcher

R¹, R², X, Y und n die in der Beschreibung angegebene Bedeutung besitzen.

Sie werden erhalten, wenn man Oxime der Formel (II)

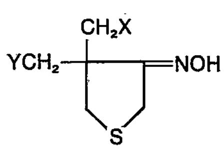

(II)

in welcher

X und Y die in der Beschreibung angegebene Bedeutung besitzen,

a) mit Carbamoylierungsmitteln umsetzt,

b) gegebenenfalls die nach dem Verfahren (a) erhältlichen Carbamoyloximino-tetrahydrothiophene oxidiert oder

c) gegebenenfalls die nach Verfahren (a) und (b) erhältlichen Carbamoyloximino-tetrahydrothiophene mit Sulfenchloriden bzw. -bromiden umsetzt.

Die neuen substituierten Carbamoyloximino-tetrahydrothiophene der Formel (I) weisen starke schädlingsbekämpfende Eigenschaften auf.

EP 0 063 742 A2

0063742

BAYER AKTIENGESELLSCHAFT
Zentralbereich
Patente, Marken und Lizenzen

5090 Leverkusen, Bayerwerk
Rt/W
Ib

Substituierte Carbamoyloximino-tetrahydrothiophene, Verfahren zu ihrer Herstellung und ihre Verwendung als Schädlingsbekämpfungsmittel

Die vorliegende Erfindung betrifft neue substituierte Carbamoyloximino-tetrahydrothiophene sowie deren Derivate, mehrere Verfahren zu ihrer Herstellung und ihre Verwendung als Schädlingsbekämpfungsmittel.

Es ist bereits bekannt geworden, daß 1-Thia-3-aminocarbonyl-oximino-cyclopentane, wie z.B. 1-Thia-4,4-dimethyl-3-methyl-aminocarbonyl-oximino-cyclopentan, pestizide Eigenschaften besitzen (vergleiche DE-AS 2 033 454). Deren Wirkung ist jedoch, insbesondere bei niedrigen Aufwandmengen, nicht immer ganz befriedigend.

1. Es wurden neue substituierte Carbamoyloximino-tetrahydrothiophene sowie deren Derivate der allgemeinen Formel (I)

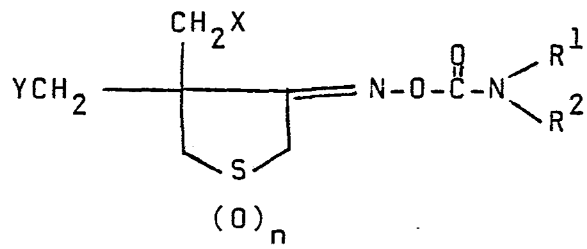

(I)

in welcher

$R^1$ für Alkyl, Alkoxyalkyl, Alkenyl oder Alkinyl steht,

$R^2$ für Wasserstoff, Alkyl oder die Gruppen $-SR^3$ und $-S_2R^6$ steht, wobei

$R^3$ für Alkyl, Halogenalkyl, gegebenenfalls substituiertes Phenyl, Alkoxycarbonyl, die Gruppierung $-NR^4R^5$, sowie für den gleichen Rest steht, an den die Gruppe $-SR^3$ gebunden ist,

$R^4$ für Alkyl, Alkoxycarbonyl oder für gegebenenfalls substituiertes Phenylsulfonyl steht,

X für Wasserstoff, Halogen oder die Gruppierungen $-OR^5$ und $-SR^5$ steht,

Y für Halogen oder die Gruppierungen $-OR^5$ und $-SR^5$ steht,

$R^5$ für Alkyl steht,

$R^6$ für Alkyl oder gegebenenfalls substituiertes Phenyl steht und

n für die Zahlen o,1 oder 2 steht,

gefunden.

Die Verbindungen der Formel (I) können in der syn- und anti-Form vorliegen; vorwiegend fallen sie als Gemische beider Formen an.

2. Weiterhin wurde gefunden, daß die substituierten Carbamoyloximino-tetrahydrothiophene der Formel (I)

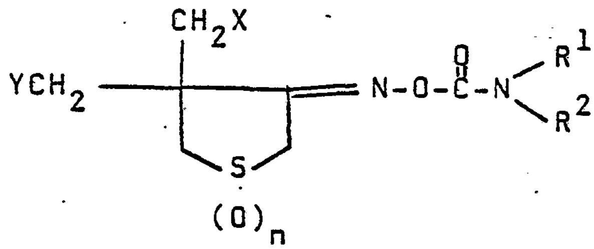

(I)

in welcher

$R^1$ für Alkyl, Alkoxyalkyl, Alkenyl oder Alkinyl steht,

$R^2$ für Wasserstoff, Alkyl oder die Gruppen $-SR^3$ und $-S_2R^6$ steht, wobei

$R^3$ für Alkyl, Halogenalkyl, gegebenenfalls substituiertes Phenyl, Alkoxycarbonyl, die Gruppierung $-NR^4R^5$, sowie für den gleichen Rest steht, an den die Gruppe $-SR^3$ gebunden ist,

$R^4$ für Alkyl, Alkoxycarbonyl oder für gegebenenfalls substituiertes Phenylsulfonyl steht,

X für Wasserstoff, Halogen oder die Gruppierungen $-OR^5$ und $-SR^5$ steht,

Y für Halogen oder die Gruppierungen $-OR^5$ und $-SR^5$ steht,

$R^5$ für Alkyl steht,

$R^6$ für Alkyl oder gegebenenfalls substituiertes Phenyl steht und

n für die Zahlen o,1 oder 2 steht,

zu erhalten sind, wenn man Oxime der Formel (II)

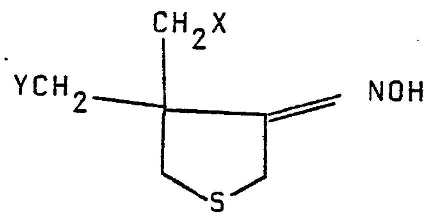

(II)

in welcher

X und Y die oben angegebene Bedeutung haben,

a) mit Carbamoylierungsmitteln umsetzt, bevorzugt

$a_1$) mit Isocyanaten der Formel (III)

$$R^1-N=C=O \qquad (III)$$

in welcher

$R^1$ die oben angegebene Bedeutung hat,

in Gegenwart eines Verdünnungsmittels und gegebenenfalls in Gegenwart eines Katalysators umsetzt, oder

$a_2$) mit Carbamoylhalogeniden der Formel (IV)

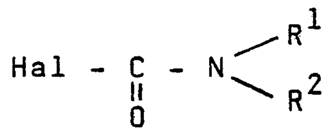

(IV)

in welcher

$R^1$ und $R^2$ die oben angegebene Bedeutung haben und

Hal für Fluor, Chlor oder Brom steht,

in Gegenwart eines Verdünnungsmittels und eines Säurebindemittels umsetzt, oder

b) gegebenenfalls die nach dem Verfahren (a) erhältlichen Carbamoyloximino-tetrahydrothiophene der Formel

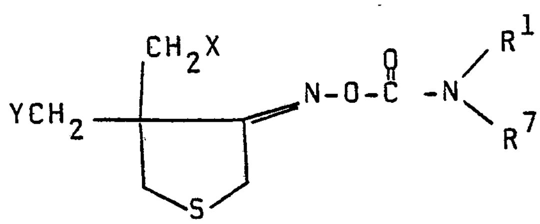

(V)

in welcher

$R^1$, X und Y die oben angegebene Bedeutung haben und

$R^7$ für Wasserstoff oder Alkyl mit 1 bis 4 Kohlenstoffatomen steht,

oxidiert, wobei je nach verwendetem Oxidationsmittel Verbindungen der Formel (I) mit n=1 oder n= 2 erhalten werden, oder

c) gegebenenfalls die nach Verfahren (a) und (b) erhältlichen Carbamoyloximino-tetrahydrothiophene der Formel (Ia)

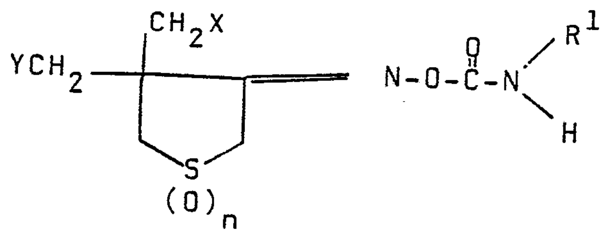 (Ia)

in welcher

$R^1$, X, Y und n die oben angegebene Bedeutung haben,

mit Sulfenchloriden bzw. -bromiden der Formel (VI)

$$Hal' - S - R^3 \qquad (VI)$$

in welcher

$R^3$ die oben angegebene Bedeutung hat und

Hal' für Chlor oder Brom steht,

in Gegenwart eines Verdünnungsmittels und eines Säurebindemittels umsetzt.

3. Es wurden ferner die neuen Oxime der Formel II gefunden,

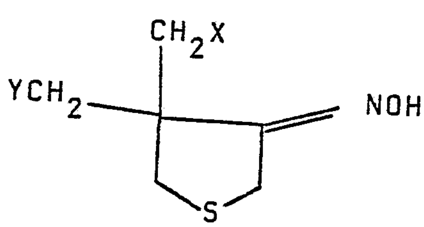 (II)

in welcher
X und Y die oben angegebene Bedeutung haben.

4. Es wurde ferner ein Verfahren zur Herstellung der Oxime der Formel (II)

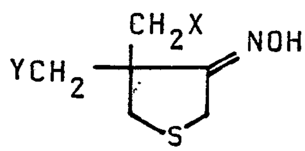 (II)

gefunden, das dadurch gekennzeichnet ist, daß man Ketone der Formel (VII)

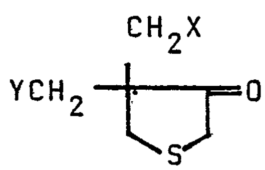 (VII)

in welcher
X und Y die oben angegebene Bedeutung haben,
mit Hydroxylamin in Gegenwart eines Lösungsmittels, bei Temperaturen zwischen 20 und 100°C, umsetzt.

5. Es wurden ferner die neuen Ketone der Formel (VII) gefunden

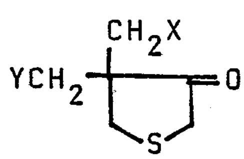 (VII)

in welcher
X und Y die oben angegebene Bedeutung besitzen.

6. Es wurde ferner ein Verfahren zur Herstellung der Ketone der Formel (VII)

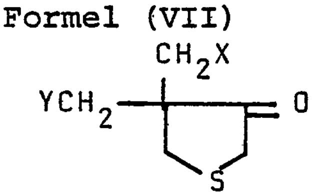 (VII)

gefunden, das dadurch gekennzeichnet ist, daß man Halogen-ketone der Formel (VIII)

$$Hal'' - CH_2 - \underset{CH_2Y}{\overset{CH_2X}{C}} - CO - CH_2 - Hal' \qquad \text{(VIII)}$$

in welcher

Hal', X und Y die oben angegebene Bedeutung haben und

Hal" für Chlor oder Brom steht,

mit Schwefelwasserstoff oder Metallhydrogensulfiden, in Gegenwart eines organischen Lösungsmittels und in Gegenwart einer Base, umsetzt.

Die neuen substituierten Carbamoyloximino-tetrahydrothiophene der Formel (I) weisen starke schädlingsbekämpfende, insbesondere insektizide und akarizide Eigenschaften auf. Die Verbindungen wirken dabei auch wurzelsystemisch. Überraschenderweise zeigen die erfindungsgemäßen Verbindungen dabei eine höhere Wirkung als das bekannte 1-Thia-4,4-dimethyl-3-methyl-aminocarbonyl-oximino-cyclopentan, welches chemisch und wirkungsmäßig die naheliegendste Verbindung ist. Die erfindungsgemäßen Wirkstoffe stellen somit eine Bereicherung der Technik dar.

Die erfindungsgemäßen substituierten Carbamoyloximino-tetrahydrothiophene sind durch die Formel (I) allgemein definiert. In dieser Formel steht $\underline{R}^1$ vorzugsweise für geradkettiges oder verzweigtes Alkyl mit 1 bis 4 Kohlenstoffatomen, Alkoxyalkyl mit 1 bis 4 Kohlenstoffatomen in jedem Alkylteil, Alkenyl und Alkinyl mit jeweils 2 bis 4 Kohlenstoffatomen. $\underline{R}^2$ steht vorzugsweise für Wasserstoff, geradkettiges oder verzweigtes Alkyl mit 1 bis 4 Kohlenstoffatomen und die Gruppen $-SR^3$ oder $-S_2R^6$. $\underline{R}^3$ steht vorzugsweise für geradkettiges oder verzweigtes Alkyl mit 1 bis 4 Kohlenstoffatomen, Halogenalkyl mit 1 bis 2 Kohlenstoff- und bis zu 5 Halogenatomen, wie insbesondere Fluor- und Chloratomen, gegebenenfalls substituiertes Phenyl, wobei als Substituenten vorzugsweise genannt seien: Halogen, insbesondere Fluor, Chlor oder Brom, Alkyl mit 1 bis 2 Kohlenstoffatomen und Halogenalkyl mit 1 bis 2 Kohlenstoff- und bis zu 5 Halogenatomen, wie insbesondere Fluor- und Chloratomen, sowie vorzugsweise für Alkoxycarbonyl mit 1 bis 4 Kohlenstoffatomen im Alkylteil.

$\underline{R}^3$ steht außerdem vorzugsweise für die Gruppierung $-NR^5R^4$, sowie für den gleichen Rest, an den die Gruppe $-SR^3$ gebunden ist. $\underline{R}^4$ steht vorzugsweise für

Alkyl mit 1 bis 4 Kohlenstoffatomen, Alkoxycarbonyl mit 1 bis 4 Kohlenstoffatomen im Alkylteil sowie für gegebenenfalls substituiertes Phenylsulfonyl, wobei als Phenylsubstituenten vorzugsweise die bei $\underline{R}^3$ bereits genannten infrage kommen. $\underline{X}$ steht vorzugsweise für Wasserstoff, Fluor, Chlor, Brom sowie die Gruppierungen $-OR^5$ und-$SR^5$. $\underline{Y}$ steht vorzugsweise für Fluor, Chlor, Brom sowie die Gruppierungen $-OR^5$ und $-SR^5$. $\underline{R}^5$ steht vorzugsweise für geradkettiges oder verzweigtes Alkyl mit 1 bis 4 Kohlenstoffatomen. $\underline{R}^6$ steht vorzugsweise für geradkettiges oder verzweigtes Alkyl mit 1 bis 4 Kohlenstoffatomen sowie für gegebenenfalls substituiertes Phenyl, wobei als Substituenten vorzugsweise die bei $R^3$ bereits vorzugsweise genannten Phenylsubstituenten infrage kommen. Der Index $\underline{n}$ steht vorzugsweise für die Zahlen 0,1 und2.

Ganz besonders bevorzugt sind diejenigen substituierten Carbamoyloximino-tertahydrothiophene der Formel (I), in denen $\underline{R}^1$ für Methyl, Ethyl, Methoxymethyl oder Allyl steht; $\underline{R}^2$ für Wasserstoff, Methyl, Ethyl oder die Gruppen $-SR^3$ und $-S_2R^6$ steht; $\underline{R}^3$ für Methyl, Trifluormethyl, Chlordifluormethyl, Dichlorfluormethyl, Trichlormethyl, gegebenenfalls durch Chlor oder Trifluormethyl substituiertes Phenyl, Methoxycarbonyl, Ethoxycarbonyl und die Gruppierung $-N(CH_3)R^4$ steht, oder $\underline{R}^3$ für den gleichen Rest steht, an den die Gruppe $-SR^3$ gebunden ist; $\underline{R}^4$ für Methyl, Ethyl, Methoxycarbonyl, Ethoxycarbonyl und gegebenenfalls durch Chlor oder Methyl substituiertes Phenylsulfonyl steht; $\underline{X}$, $\underline{Y}$ und der Index $\underline{n}$ die oben angegebene Bedeutung haben, $\underline{R}^5$ für Methyl oder Ethyl steht, und $\underline{R}^6$ für Methyl, Ethyl und gegebenenfalls durch Chlor oder Trifluormethyl

substituiertes Phenyl steht.

Im einzelnen seien außer den bei den Herstellungsbeispielen genannten Beispiele folgende Verbindungen der Formel (I) genannt:

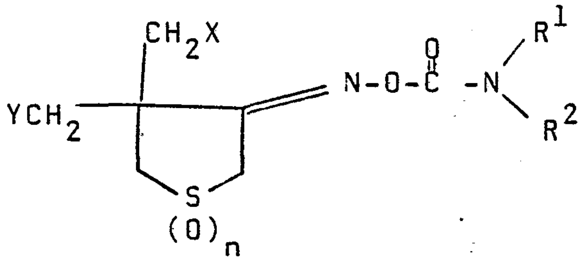

(I)

| X | Y | $R^1$ | $R^2$ | n |
|---|---|---|---|---|
| H | F | $CH_3$ | $-SCCl_2F$ | 0 |
| H | Cl | $CH_3$ | $-SCCl_2F$ | 0 |
| H | Br | $CH_3$ | $-SCCl_2F$ | 0 |
| F | F | $CH_3$ | $-SCCl_2F$ | 0 |
| Cl | Cl | $CH_3$ | $-SCCl_2F$ | 0 |
| Br | Br | $CH_3$ | $-SCCl_2F$ | 0 |
| H | F | $CH_3$ | $-SCCl_3$ | 0 |
| H | Cl | $CH_3$ | $-SCCl_3$ | 0 |
| H | Br | $CH_3$ | $-SCCl_3$ | 0 |

| X | Y | $R^1$ | $R^2$ | n |
|---|---|---|---|---|
| Cl | Cl | $CH_3$ | $-SCCl_3$ | 0 |
| Br | Br | $CH_3$ | $-SCCl_3$ | 0 |
| F | F | $CH_3$ | $-SCCl_3$ | 0 |
| H | F | $CH_3$ | H | 0 |
| H | Cl | $CH_3$ | H | 0 |
| H | Br | $CH_3$ | H | 0 |
| F | F | $CH_3$ | H | 0 |
| Cl | Cl | $CH_3$ | H | 0 |
| Br | Br | $CH_3$ | H | 0 |
| H | Cl | $CH_3$ | $-SCH_3$ | 0 |
| H | Cl | $CH_3$ | 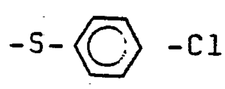 | 0 |
| H | Cl | $CH_3$ | $-S-N(CH_3)-COOC_2H_5$ | 0 |
| H | Cl | $CH_3$ | $-S-N(CH_3)_2$ | 0 |
| H | Cl | $CH_3$ | 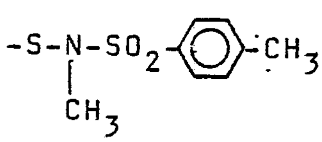 | 0 |
| H | Cl | $CH_3$ | $-S-S-CH_3$ | 0 |
| H | Cl | $CH_3$ | dimer | 0 |

Verwendet man beispielsweise 4-Chlormethyl-3-hydroximino-4-methyl-tetrahydrothiophen und Methylisocyanat als Ausgangsstoffe, so kann der Reaktionsablauf durch das folgende Formelschema wiedergegeben werden (Verfahren $a_1$):

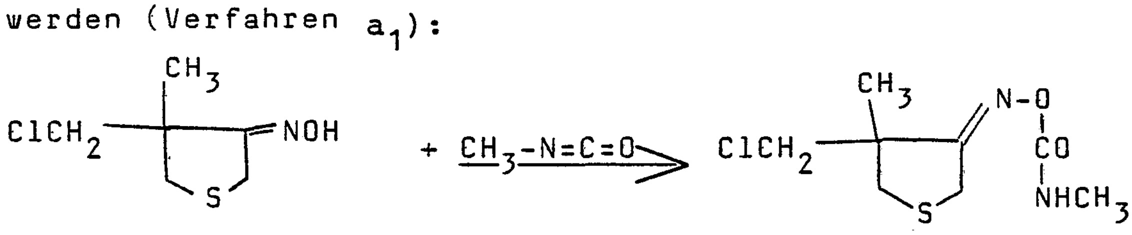


Verwendet man beispielsweise 4-Chlormethyl-3-hydroximino-4-methyl-tetrahydrothiophen und N-Methyl-N-trichlormethyl-mercapto-carbamoylfluorid als Ausgangsstoffe, so kann der Reaktionsablauf durch das folgende Formelschema wiedergegeben werden (Verfahren $a_1$):

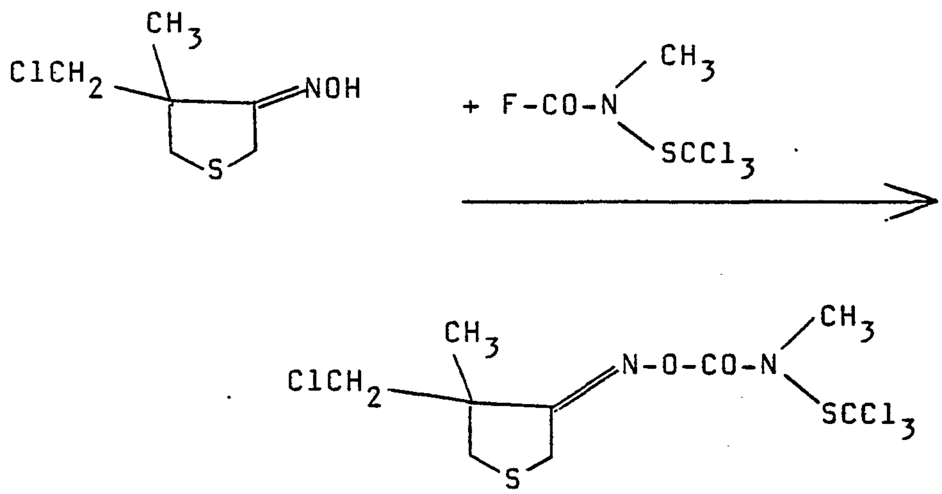

Verwendet man beispielsweise 2 Mol 4-Chlormethyl-3-hydroximino-4-methyl-tetrahydrothiophen und 1 Mol N,N'-Bis-(fluorcarbonyl)-thio-bis-methylamin als Ausgangsstoffe, so kann der Reaktionsablauf durch das folgende Formelschema wiedergegeben werden ( Verfahren $a_2$):

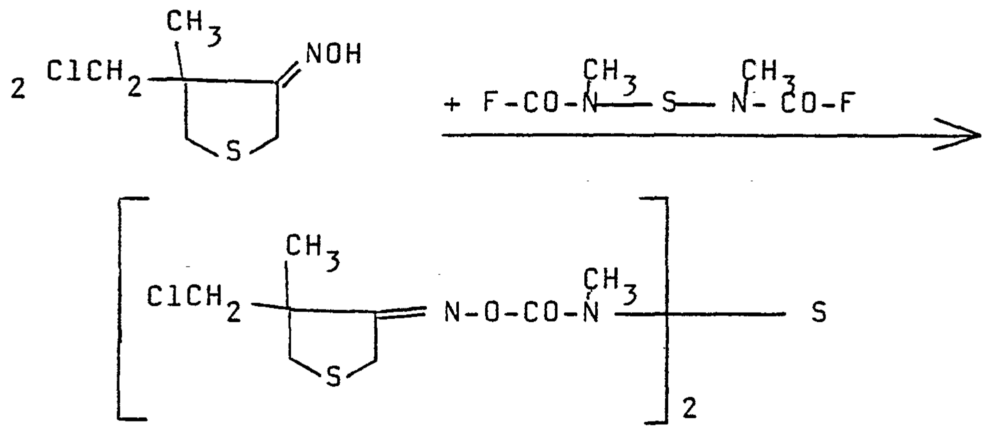

Verwendet man beispielsweise 4-Chlormethyl-4-methyl-3-methylcarbamoyl-oximino-tertahydrothiophen als Ausgangsstoff und Wasserstoffperoxid als Oxidationsmittel, so kann der Reaktionsablauf durch das folgende Formelschema wiedergegeben werden (Verfahren b):

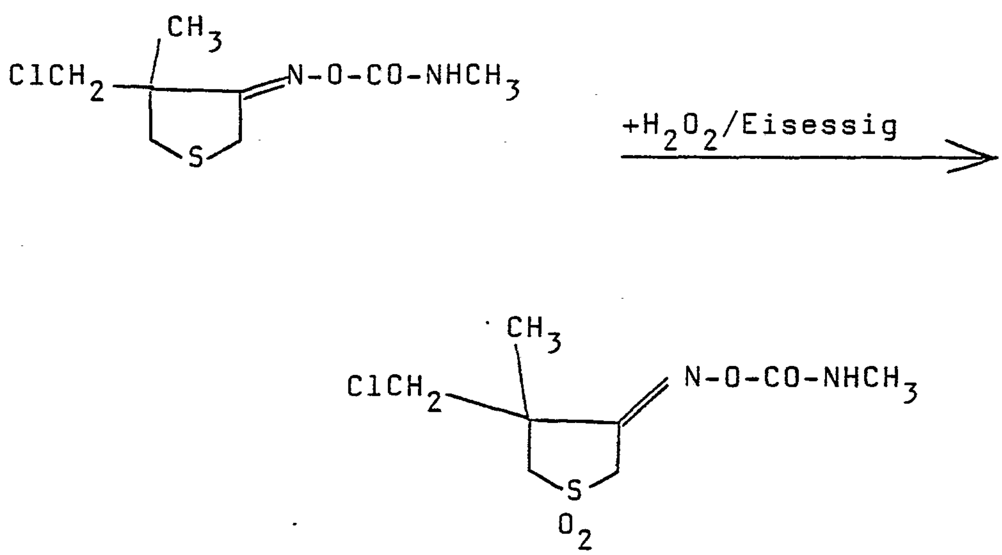


Verwendet man beispielsweise 4-Chlormethyl-4-methyl-3-methylcarbamoyl-oximino-tetrahydrothiophen und 4-Chlorphenyl-sulfenylchlorid als Ausgangsstoffe, so kann der Reaktionsablauf durch das folgende Formelschema wiedergegeben werden (Verfahren c):

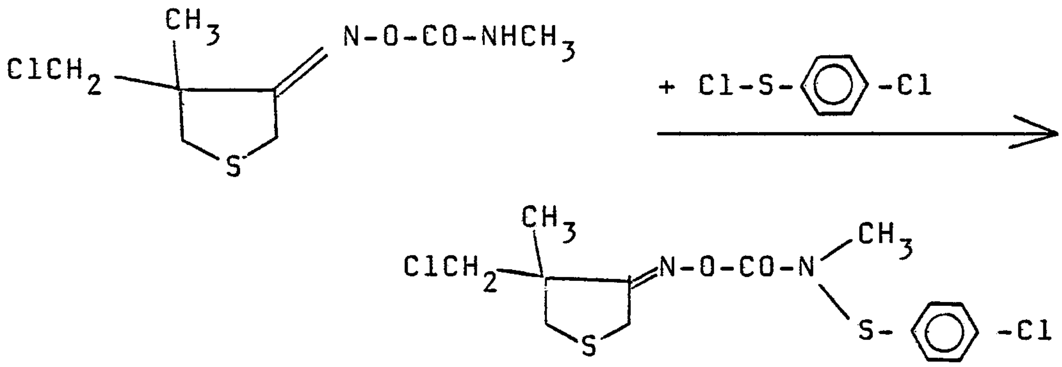

Die für die Verfahren ($a_1$) und ($a_2$) als Ausgangsstoffe zu verwendenden Oxime sind durch die Formel (II) allgemein definiert. In dieser Formel stehen X und Y vorzugsweise für die Reste, die bei den Verbindungen der Formel (I) bereits vorzugsweise genannt wurden.

Die Oxime der Formel (II) sind noch nicht bekannt; sie können jedoch erhalten werden, indem man Ketone der Formel (VII)

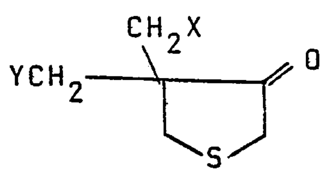

(VII)

in welcher

X und Y die oben angegebene Bedeutung haben,

mit Hydroxylamin in Gegenwart eines Lösungsmittels, vorzugsweise Alkohole bzw. wäßrige Alkohole, bei Temperaturen zwischen 20 und 100 °C, vorzugsweise zwischen 50 und 80°C, umsetzt. Dabei wird das Hydroxylamin vorzugsweise in Form seiner Salze, insbesondere als Hydrochlorid, in Gegenwart eines Säurebindemittels, wie beispielsweise Natriumcarbonat, eingesetzt. Die Isolierung der Verbindungen

der Formel (II) erfolgt dadurch, daß man das während der Umsetzung entstehende Produkt nach Abdestillieren des Lösungsmittels nach üblichen Methoden aufarbeitet (vergleiche auch die Herstellungsbeispiele).

Die Ketone der Formel (VII) können erhalten werden, indem man Halogen-ketone der Formel (VIII)

$$\begin{array}{c} \quad\quad\quad\quad CH_2X \\ \quad\quad\quad\quad | \\ Hal''-CH_2 - C - CO - CH_2 - Hal' \\ \quad\quad\quad\quad | \\ \quad\quad\quad\quad CH_2Y \end{array} \qquad (VIII)$$

in welcher

Hal',X und Y die oben angegebene Bedeutung haben und

Hal" für Chlor oder Brom steht,

mit Schwefelwasserstoff oder Metallhydrogensulfiden, insbesondere Alkalimetall- und Erdalkalimetall-hydrogensulfiden, in Gegenwart eines organischen Lösungsmittels, wie beispielsweise Methanol, und in Gegenwart einer Base, wie beispielsweise Natriummethylat, bei Temperaturen zwischen 0 und 100°C umsetzt (vergleiche auch die Herstellungsbeispiele).

Die Halogen-ketone der Formel (VIII) sind teilweise bekannt (vergleiche z.B. DE-OS 26 32 603 und DE-OS 29 18 895), bzw. können sie in allgemein bekannter Art und Weise erhalten werden.

Die außerdem für das erfindungsgemäße Verfahren $(a_1)$ als Ausgangsstoffe benötigten Isocyanate sind durch die Formel (III) allgemein definiert. In dieser Formel steht $\underline{R}^1$ vorzugsweise für die Reste, die bei den Verbindungen der Formel (I) bereits vorzugsweise genannt wurden.

Die Isocyanate der Formel (III) sind bekannt oder lassen sich nach allgemein üblichen und bekannten Verfahren herstellen.

Die außerdem für das erfindungsgemäße Verfahren $(a_2)$ als Ausgangsstoffe benötigten Carbamoylhalogenide sind durch die Formel (IV) allgemein definiert. In dieser Formel stehen $\underline{R}^1$ und $\underline{R}^2$ vorzugsweise für die Reste, die bei den Verbindungen der Formel (I) bereits vorzugsweise genannt wurden.

Die Carbamoylhalogenide der Formel (IV) sind bekannt oder lassen sich nach allgemein üblichen und bekannten Verfahren herstellen, z.B. durch Umsetzung von Aminen mit Phosgen (diese Verfahren sind aus den allgemeinen Lehrbüchern der organischen Chemie bekannt) oder durch Umsetzung der entsprechenden Carbaminsäurehalogenide mit entsprechenden Sulfenchloriden (vergleiche hierzu auch die Angaben in DE-AS 1 297 095, DE-OS 23 57 930 und 24 09 463 sowie US-Patentschrift 3 939 192).

Die weiterhin für das Verfahren (c) als Ausgangsstoffe benötigten Sulfenchloride bzw. -halogenide sind durch die Formel (VI) allgemein definiert. In dieser Formel steht $\underline{R}^3$ vorzugsweise für die Reste, die bei den Verbindungen der Formel (I) bereits vorzugsweise genannt wurden.

Die Sulfenchloride bzw. -bromide sind allgemein bekannte Verbindungen der organischen Chemie.

Als Verdünnungsmittel kommen für die Umsetzung gemäß Verfahrensvarianten ($a_1$), ($a_2$) und (c) vorzugsweise alle inerten organischen Lösungsmittel infrage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Formamide, wie insbesondere Dimethylformamid und halogenierte Kohlenwasserstoffe, wie Methylenchlorid, Tetrachlorkohlenstoff oder Chloroform.

Wird die Umsetzung nach Verfahren ($a_2$) und (c) in Gegenwart eines Säurebinders vorgenommen, so kann man alle üblicherweise verwendbaren anorganischen und organischen Säurebinder zugeben. Hierzu gehören vorzugsweise Natriumcarbonat, Kaliumcarbonat und Natriumhydrogencarbonat, ferner niedere tertiäre Alkylamine, Cycloalkylamine oder Aralkylamine, wie beispielsweise Triethylamin, N,N-Dimethyl-benzylamin, Dicyclohexylamin, weiterhin Pyridin und Diazabicyclooctan.

Als Katalysatoren können beim Verfahren ($a_1$) vorzugsweise verwendet werden:
tertiäre Basen, wie Triethylamin, Pyridin, Zinn-organische Verbindungen, Dibutylzinndilaurat.

Die Reaktionstemperaturen können bei der Durchführung des Verfahrens ($a_1$) in einem größeren Bereich variiert werden. Im allgemeinen arbeitet man zwischen 0 und 100°C, vorzugsweise zwischen 20 und 85°C.

Bei der Durchführung des Verfahrens ($a_1$) setzt man auf 1 Mol der Verbindung der Formel (II) 1 bis 2 Mol Iso-

cyanat der Formel (III) ein. Zur Isolierung der Verbindungen der Formel (I) wird das Lösungsmittel abdestilliert und der Rückstand nach üblichen Methoden aufgearbeitet.

Die Reaktionstemperaturen können bei der Durchführung des Verfahrens ($a_2$) in einem größeren Bereich variiert werden. Im allgemeinen arbeitet man zwischen 0 und 100°C, vorzugsweise zwischen 10 und 80°C.

Bei der Durchführung des Verfahrens ($a_2$) setzt man vorzugsweise auf 1 Mol der Verbindung der Formel (II) 1 bis 2 Mol, für den Fall eines dimeren Produktes 0,5 Mol, an Caramoylhalogenid der Formel (IV) und 1 bis 2 Mol Säurebinder ein. Die Isolierung der Verbindungen der Formel (I) erfolgt in allgemein üblicher und bekannter Weise.

Für das Verfahren (b) kommen als Oxidationsmittel alle üblicherweise verwendbaren anorganischen und organischen Oxidationsmittel in Betracht, wie Chlor in Wasser; Persäure, beispielsweise Metachlorperbenzoesäure; Wasserstoffperoxid in Eisessig oder Methanol, Kaliumpermanganat und Chromsäure.

Die Reaktionstemperaturen können bei der Oxidation gemäß Verfahren (b) in einem größeren Bereich variiert werden. Im allgemeinen arbeitet man zwischen etwa -30 bis + 100°C, vorzugsweise bei -10 bis +80°C.

Bei der Durchführung der Oxidation gemäß Verfahren (b) setzt man auf 1 Mol der Verbindung der Formel (V) etwa 1 bis 4 Mol Oxidationsmittel ein. Bei der Anwendung von 1 Mol Oxidationsmittel, wie Metachlorperbenzoesäure in Methylenchlorid oder Wasserstoffperoxid in Acetanhydrid

bei Temperaturen zwischen -10 bis + 10°C entstehen vorzugsweise die erfindungsgemäßen Verbindungen der Formel (I) mit n = 1. Bei Überschuß an Oxidationsmittel und höheren Temperaturen (10 bis 80°C) enstehen vorzugsweise die erfindungsgemäßen Verbindungen der Formel (I) mit n = 2.

Zur Isolierung der Oxidationsprodukte wird das Reaktionsgemisch entweder auf Eiswasser gegeben und abgesaugt, der zurückbleibende Niederschlag wird gegebenenfalls gewaschen und getrocknet, oder die Reaktionslösung wird auf pH 7 bis 8 gestellt, mit einem organischen Lösungsmittel extrahiert und die extrahierte Phase getrocknet und das Lösungsmittel abdestillert. Die Reaktionsprodukte können in beiden Fällen durch Umkristallisation oder Säulenchromatographie gereinigt werden.

Die Reaktionstemperaturen können bei der Durchführung des Verfahrens (c) in einem größeren Bereich variiert werden. Im allgemeinen arbeitet man zwischen 0 und 100°C, vorzugsweise zwischen 10 und 50°C.

Bei der Durchführung des Verfahrens (c) werden die Ausgangsstoffe vorzugsweise in molaren Mengen eingesetzt. Die Isolierung der Verbindungen der Formel (I) erfolgt nach üblichen Methoden.

Zum Teil ist es auch möglich die einzelnen Stufen der Vorprodukte zur Herstellung der Oxime der Formel (II) sowie deren Umsetzung zu den erfindungsgemäßen Stoffen ohne Isolierung der jeweiligen Zwischenprodukte in einer sogenannten Eintopfreaktion durchzuführen.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp.,Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo - und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

0063742

Herstellungsbeispiele :

Beispiel 1

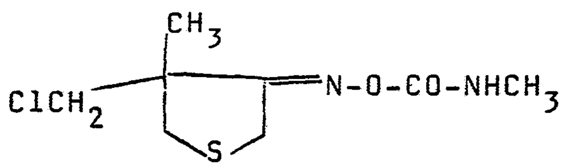

(Verfahren $a_1$)

10,2 g ( 0,057 Mol) 4-Chlormethyl-3-hydroximino-4-methyl-tetrahydrothiophen in 180 ml Toluol werden bei Raumtemperatur mit einer Lösung von 10 g Methylisocyanat in 20 ml Toluol versetzt. Man läßt 15 Stunden bei Raumtemperatur nachrühren. Danach wird das Reaktionsgemisch mit verdünnter Salzsäure versetzt, die organische Phase wird abgetrennt, mit Natriumhydrogencarbonatlösung gewaschen, über Natriumsulfat getrocknet und im Vakuum eingeengt. Man erhält 12,9g ( 96 % der Theorie) 4-Chlormethyl-4-methyl-3-methylcarbamoyloximino-tertahydrothiophen vom Schmelzpunkt 103-105°C.

Herstellung des Ausgangsproduktes

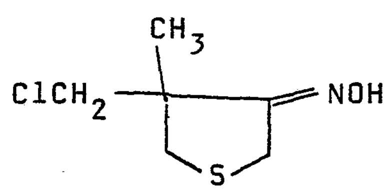

15,4g (0,094 Mol) 4-Chlormethyl-4-methyl-3-oxo-tetrahydrothiophen in 100 ml Methanol werden mit einer Lösung von 13,1 g (0,188 Mol) Hydroxylaminhydrochlorid und 15,4 g Natriumactat in 30 ml Wasser versetzt. Man läßt 15 Stunden bei 25°C nachrühren. Nach üblicher Aufarbeitung des Reaktionsgemisches und Umkristallisation aus n-Hexan erhält man 11,8g

(70 % der Theorie) 4-Chlormethyl-3-hydroximino-4-methyl-tetrahydrothiophen vom Schmelzpunkt 86-87°C.

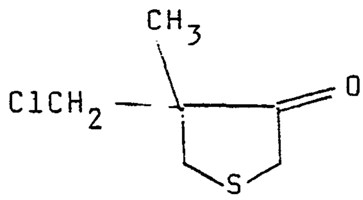

66,7g (0,27 Mol) 1-Brom-4-chlor-3-chlormethyl-3-methylbutan-2-on in 200 ml Methanol werden bei 10 - 20°C mit einer 30 %-igen Lösung von 51 g (0,27 Mol) Natriumhydrogensulfid versetzt. Man läßt das Reaktionsgemisch 2 Stunden bei 20°C nachrühren und gibt es anschließend in Wasser. Man extrahiert mit Methylenchlorid , engt ein und destilliert den Rückstand im Vakuum. Man erhält 35,4 g ( 80 % der Theorie) 4-Chlormethyl-4-methyl-3-oxo-tetrahydrothiophen vom Brechungsindex $n_D^{20}$ =1,5149.

Beispiel 2

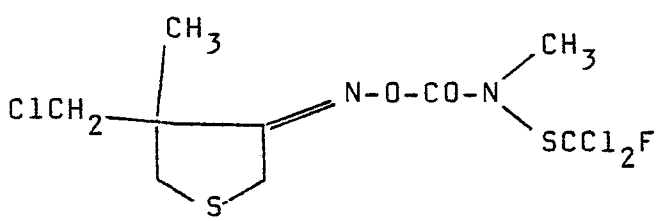

(Verfahren $a_2$)
27,4 g(0,153 Mol) 4-Chlormethyl-3-hydroximino-4-methyl-tetrahydrothiophen und 15,5g (0,153 Mol) Triethylamin in 160 ml Toluol werden bei Raumtemperatur mit 32,1g (0,153 Mol) N-Dichlorfluormethylmercapto-N-methylcarbamoylfluorid in 80 ml Toluol versetzt. Man läßt 4 Stunden nachrühren bei 40°C. Nach üblicher Aufarbeitung erhält man 48,3g (85 % der Theorie) 4-Chlormethyl-4-methyl-3-(N-dichlorfluormethylmercapto-N-methyl)-carbamoyl-oximino-tetrahydrothiophen vom Brechungsindex $n_D^{20}$ =1,5460.

Analog werden die folgenden Verbindungen erhalten:

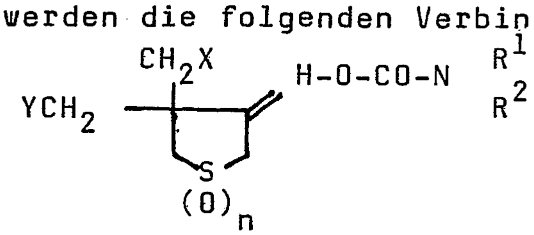

| Beispiel Nr. | X | Y | n | $R^1$ | $R^2$ | Schmelzpunkt (°C) |
|---|---|---|---|---|---|---|
| 3 | H | Cl | 0 | $CH_3$ | dimer | 40 |
| 4 | H | Cl | 0 | $CH_3$ | $SCCl_3$ | 94 |
| 5, | H | Cl | 0 | $CH_3$ | $-SN(CH_3)_2$ | oil |

Anwendungsbeispiele

In den nachfolgenden Beispielen wird die nachstehende Verbindung als Vergleichssubstanz eingesetzt:

(A) 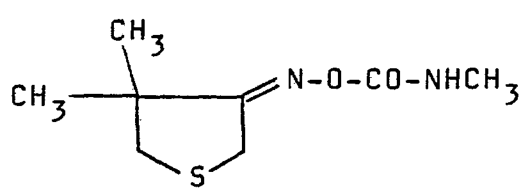

0063742

Beispiel A

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1

0063742

Beispiel B

Myzus-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 2.

Beispiel C

Tetranychus-Test (resistent)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 2.

## Patentansprüche

1) Substituierte Carbamoyloximino-tetrahydrothiophene sowie deren Derivate der allgemeinen Formel (I)

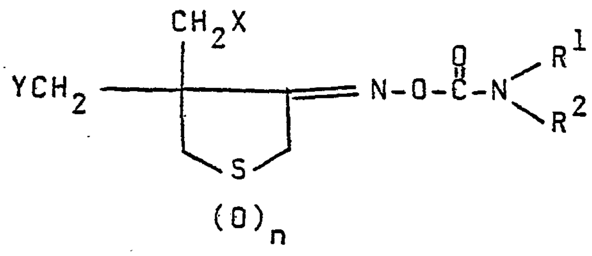

(I)

in welcher

$R^1$ für Alkyl, Alkoxyalkyl, Alkenyl oder Alkinyl steht,

$R^2$ für Wasserstoff, Alkyl oder die Gruppen $-SR^3$ und $-S_2R^6$ steht, wobei

$R^3$ für Alkyl, Halogenalkyl, gegebenenfalls substituiertes Phenyl, Alkoxycarbonyl, die Gruppierung $-NR^4R^5$, sowie für den gleichen Rest steht, an den die Gruppe $-SR^3$ gebunden ist,

$R^4$ für Alkyl, Alkoxycarbonyl oder für gegebenenfalls substituiertes Phenylsulfonyl steht,

X für Wasserstoff, Halogen oder die Gruppierungen $-OR^5$ und $-SR^5$ steht,

Y für Halogen oder die Gruppierungen $-OR^5$ und $-SR^5$ steht,

$R^5$ für Alkyl steht,

$R^6$ für Alkyl oder gegebenenfalls substituiertes Phenyl steht und

n für die Zahlen o,1 oder 2 steht.

0063742

2) Verfahren zur Herstellung von substituierten Carbamoyloximino-tetrahydrothiophene und deren Derivaten der Formel (I)

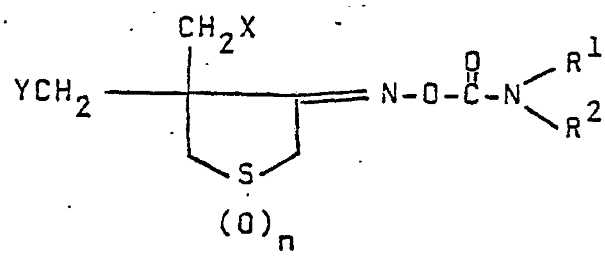

(I)

in welcher

$R^1$ für Alkyl, Alkoxyalkyl, Alkenyl oder Alkinyl steht,

$R^2$ für Wasserstoff, Alkyl oder die Gruppen $-SR^3$ und $-S_2R^6$ steht, wobei

$R^3$ für Alkyl, Halogenalkyl, gegebenenfalls substituiertes Phenyl, Alkoxycarbonyl, die Gruppierung $-NR^4R^5$, sowie für den gleichen Rest steht, an den die Gruppe $-SR^3$ gebunden ist,

$R^4$ für Alkyl, Alkoxycarbonyl oder für gegebenenfalls substituiertes Phenylsulfonyl steht,

X für Wasserstoff, Halogen oder die Gruppierungen $-OR^5$ und $-SR^5$ steht,

Y für Halogen oder die Gruppierungen $-OR^5$ und $-SR^5$ steht,

$R^5$ für Alkyl steht,

$R^6$ für Alkyl oder gegebenenfalls substituiertes Phenyl steht und

n für die Zahlen 0, 1 oder 2 steht,

dadurch gekennzeichnet, daß man Oxime der Formel (II)

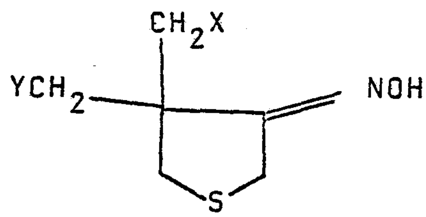

(II)

in welcher

X und Y die oben angegebene Bedeutung haben,

a) mit Carbamoylierungsmitteln umsetzt,

b) gegebenenfalls die nach dem Verfahren (a) erhältlichen Carbamoyloximino-tetrahydrothiophene der Formel

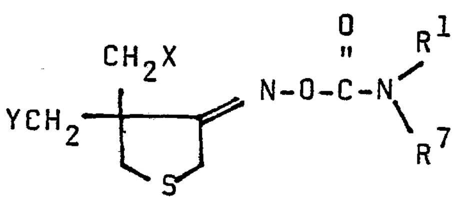

(V)

in welcher

$R^1$, X und Y die oben angegebene Bedeutung haben und

$R^7$ für Wasserstoff oder Alkyl mit 1-4 Kohlenstoffatomen steht,

oxidiert, wobei je nach verwendetem Oxidationsmittel Verbindungen der Formel (I) mit n = 1 oder n = 2 erhalten werden, oder

c) gegebenenfalls die nach Verfahren (a) oder (b) erhältlichen Carbamoyloximino-tetrahydrothiophene der Formel (Ia)

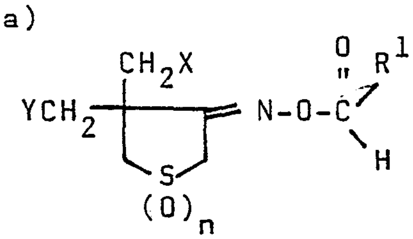

in welcher
$R^1$, X, Y und n die oben angegebene Bedeutung haben,

mit Sulfenchloriden bzw. -bromiden der Formel (VI)

$$Hal'-S-R^3 \qquad (VI)$$

in welcher
$R^3$ die oben angegebene Bedeutung hat und
Hal' für Chlor oder Brom steht,

in Gegenwart eines Verdünnungsmittels und eines Säurebindemittels umsetzt.

3) Oxime der Formel (II)

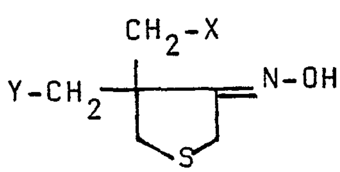

(II)

in welcher

X für Wasserstoff, Halogen oder die Gruppierungen $-OR^5$ und $-SR^5$ steht,

Y für Halogen oder die Gruppierungen $-OR^5$ und $-SR^5$ steht und

$R^5$ für Alkyl steht.

4. Verfahren zur Herstellung der Oxime der Formel (II)

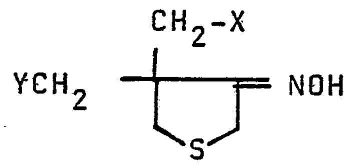

(II)

dadurch gekennzeichnet, daß man Ketone der Formel (VII)

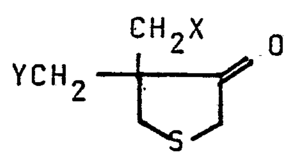

(VII)

in welcher

X für Wasserstoff, Halogen oder die Gruppierungen $-OR^5$ und $-SR^5$ steht,

Y für Halogen oder die Gruppierungen $-OR^5$ und $-SR^5$ steht,

$R^5$ für Alkyl steht,

mit Hydroxylamin in Gegenwart eines Lösungsmittels bei Temperaturen zwischen 20 und 100°C umsetzt.

5. Ketone der Formel (VII)

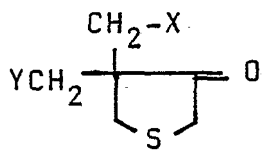

(VII)

in welcher

X für Wasserstoff, Halogen oder die Gruppierungen $-OR^5$ und $-SR^5$ steht,

Y für Halogen oder die Gruppierungen $-OR^5$ und $-SR^5$ steht,

$R^5$ für Alkyl steht.

6. Verfahren zur Herstellung der Ketone der Formel (VII)

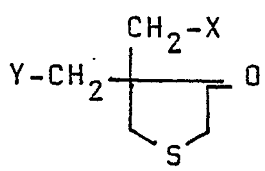

(VII)

in welcher

X für Wasserstoff, Halogen oder die Gruppierungen $-OR^5$ und $-SR^5$,

Y für Halogen oder die Gruppierungen $-OR^5$ und $-SR^5$ und

$R^5$ für Alkyl steht,

das dadurch gekennzeichnet ist, daß man Halogenketone der Formel (VIII)

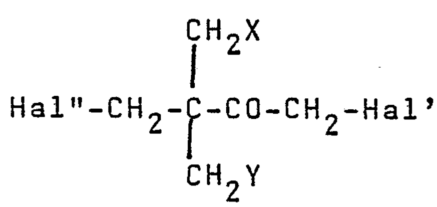

(VIII)

in welcher
X und Y die oben angegebene Bedeutung haben und
Hal' und Hal" für Chlor oder Brom steht,

mit Schwefelwasserstoff oder Metallhydrogensulfiden in Gegenwart eines organischen Lösungsmittels und in Gegenwart einer Base umsetzt.

7. Substituierte Carbamoyloximinotetrahydrothiophene der Formel (I) gemäß Anspruch 1,
wobei
$R^1$ für geradkettiges oder verzweigtes Alkyl mit 1 bis 4 Kohlenstoffatomen, Alkoxyalkyl mit 1 bis 4 Kohlenstoffatomen in jedem Alkylteil, Alkenyl und Alkinyl mit jeweils 2 bis 4 Kohlenstoffatomen steht,
$R^2$ für Wasserstoff, geradkettiges oder verzweigtes Alkyl mit 1 bis 4 Kohlenstoffatomen und die Gruppen $-SR^3$ oder $-S_2R^6$ steht,
$R^3$ für geradkettiges oder verzweigtes Alkyl mit 1 bis 4 Kohlenstoffatomen, Halogenalkyl mit 1 bis 2 Kohlenstoff- und bis zu 5 Halogenatomen, wie insbesondere Fluor- und Chloratomen, gegebenenfalls substituiertes Phenyl, wobei als Substituenten genannt seien:
Halogen, Alkyl mit 1 bis 2 Kohlenstoffatomen und Halogenalkyl mit 1 bis 2 Kohlenstoff- und

bis zu 5 Halogenatomen, für Alkoxycarbonyl mit 1 bis 4 Kohlenstoffatomen im Alkylteil steht.

$R^3$ steht außerdem für die Gruppierung $-NR^5R^4$, sowie für den gleichen Rest, an den die Gruppe $-SR^3$ gebunden ist,

$R^4$ steht für Alkyl mit 1 bis 4 Kohlenstoffatomen, Alkoxycarbonyl mit 1 bis 4 Kohlenstoffatomen im Alkylteil sowie für gegebenenfalls substituiertes Phenylsulfonyl, wobei als Phenylsubstituenten die bei $R^3$ bereits genannten infrage kommen,

X steht für Wasserstoff, Fluor, Chlor, Brom sowie die Gruppierungen $-OR^5$ und $-SR^5$,

Y steht für Fluor, Chlor, Brom sowie die Gruppierungen $-OR^5$ und $-SR^5$,

$R^5$ steht für geradkettiges oder verzweigtes Alkyl mit 1 bis 4 Kohlenstoffatomen,

$R^6$ steht für geradkettiges oder verzweigtes Alkyl mit 1 bis 4 Kohlenstoffatomen sowie für gegebenenfalls substituiertes Phenyl, wobei als Substituenten die bei $R^3$ bereits genannten Phenylsubstituenten infrage kommen.

Der Index n steht für die Zahlen 0, 1 und 2.

8. Substituierte Carbamoyloximino-tetrahydrothiophene der Formel (I) gemäß Anspruch 1, wobei
   $R^1$ für Methyl, Ethyl, Methoxymethyl oder Allyl steht,
   $R^2$ für Wasserstoff, Methyl, Ethyl oder die Gruppen $-SR^3$ und $-S_2R^6$ steht,
   $R^3$ für Methyl, Trifluormethyl, Chlordifluormethyl, Dichlorfluormethyl, Trichlormethyl, gegebenenfalls durch Chlor oder Trifluormethyl substituiertes Phenyl, Methoxycarbonyl, Ethoxycarbonyl und die Gruppierung $-N(CH_3)R^4$ steht oder
   $R^3$ für den gleichen Rest steht, an den die Gruppe $-SR^3$ gebunden ist,
   $R^4$ für Methyl, Ethyl, Methoxycarbonyl, Ethoxycarbonyl und gegebenenfalls durch Chlor oder Methyl substituiertes Phenylsulfonyl steht,
   X, Y und der Index n die oben genannte Bedeutung haben,
   $R^5$ für methyl oder Ethyl steht und
   $R^6$ für Methyl, Ethyl und gegebenenfalls durch Chlor oder Trifluormethyl substituiertes Phenyl steht.

9. Schädlingsbekämpfungsmittel, gekennzeichnet durch einen Gehalt an mindestens einem substituierten Carbamoyloximino-tetrahydrothiophen oder einem Derivat der Formel (I).

10. Verwendung von substituierten Carbamoyloximino-tetrahydrothiophenen sowie deren Derivaten der Formel (I) zur Bekämpfung von Schädlingen.